# EUROPEAN PATENT SPECIFICATION

(11) **EP 3 490 577 B1**
(45) Date of publication and mention of the grant of the patent: **08.12.2021**
(21) Application number: 17751057.5
(22) Date of filing: 31.07.2017
(51) Int. Cl.: A61K 36/9066, A61K 31/121, A61K 31/23, A61K 31/231, A61K 31/721, A61K 31/728, A61K 33/38, A61K 9/00, A61K 9/107, A61K 9/113, A61K 47/12, A61K 47/14, A61K 47/26, A61K 47/32, A61K 47/36, A61K 8/00, A61P 17/16, A61P 17/18

(54) **SKIN COMPOSITIONS COMPRISING TURMERONES**
ZUSAMMENSETZUNG ZUR BEHANDLUNG DER HAUT ENTHALDEND TURMERONE
COMBINAISONS POUR LA PEAU CONTENANT DES TURMÉRONES

(30) Priority: 01.08.2016 PL 41816816
(43) Date of publication of application: 05.06.2019
(73) Proprietor: Adamed Pharma S.A., 05-152 Czosnow (PL)
(72) Inventor: MAJKA, Zbigniew, 39-102 Lubzina (PL)
(74) Representative: Sulikowski, Daniel
(86) International application number: PCT/EP2017/069292
(87) International publication number: WO 2018/024662

(56) References cited:
- WO-A1-2013/016257
- WO-A2-2011/014883
- US-A1- 2006 134 059
- US-A1- 2010 068 161
- TANUJA YADAV ET AL: "Anticedants and natural prevention of environmental toxicants induced accelerated aging of skin", ENVIRONMENTAL TOXICOLOGY AND PHARMACOLOGY, vol. 39, no. 1, 3 December 2014 (2014-12-03), pages 384-391, XP055412211, NL ISSN: 1382-6689, DOI: 10.1016/j.etap.2014.11.003

## Description

The invention relates to a skin composition, a spray containing the skin composition and use of the skin composition.

Radiation dermatitis is a serious adverse effect associated with treatment with ionising radiation. A radiation-induced skin reaction is manifested by a burning sensation, pruritus and sometimes pain at the irradiated site. According to RTOG/EORTC (Radiation Therapy Oncology Group/European Organization for Research and Treatment of Cancer), there are four grades of radiation-induced skin reactions.

Grade 1 involves the appearance of erythema on the skin. Dry desquamation is a symptom of grade 2. Excessive drying of the skin is associated with damage to the basal layer cells as well as with damage to sebaceous and sweat glands which provide appropriate moisture to the epidermis. Grade 3 is associated with moist desquamation. Grade 4 of radiation-induced skin reaction involves skin ulceration.

The grade of a radiation-induced skin reaction depends on the dose of radiation and on individual characteristics of the skin. More extensive skin damage occurs mainly in patients with kidney diseases, in those drinking alcohol and smoking cigarettes. The grade of a radiation-induced skin reaction also depends on age. It is higher in elderly people than in younger ones.

Treatment of radiation-induced dermatitis aims at managing inflammation and pain in or bleeding from damaged skin. Such treatment is implemented with the use of pharmacological products as well as greasy barrier ointments whose consistency makes application difficult. Cleansing of the skin affected by a radiation-induced skin reaction requires mild cleansing agents. Such products must not contain ingredients that have irritant or allergenic effects. As the barrier functions of the skin are impaired, the cleansing agents must not cause excessive drying of the skin or affect its natural pH. Skin care products should not contain fragrance compositions or colouring agents as they may cause skin irritation.

Treatment of radiation-induced skin reactions of various grades involves the use of products containing aloe vera juice, marigold extract, hyaluronic acid, glucocorticosteroids, hydrocolloid dressings and silver sulfadiazine. Therapeutic effects of aloe vera juice have not been confirmed in scientific studies. Products containing marigold extract alleviate pain, and hyaluronic acid has skin protective properties and is effective both during and after radiation. Glucocorticosteroid-containing products are used both in the prevention and treatment of inflammatory responses. However, the use of such products is associated with adverse effects, typical of this class of drugs.

The results of using hydrocolloid dressings have been inconclusive.

Silver sulfadiazine is used to prevent and treat skin infections in radiation-induced skin conditions of higher grades (3 and 4). Its anti-microbial action is related to the presence of silver ions and sulfadiazine which is a sulfonamide compound.

Various cosmetic products are also used in irradiated skin care, but since they are not indicated for treatment and/or prevention, they can be used only as auxiliary products in topical care and where skin integrity has not been compromised.

For example, there is a product commercially available in the form of a face and body cream of the following composition (INCI): Aqua, Paraffinum Liquidum, Isododecane, Pentylene Glycol, Panthenol, Glycerin, Cetyl PEG/PPG-10/1 Dimethicone, Magnesium Sulfate, Polyglyceryl-4 Isostearate, Cera Alba (Beeswax), Sorbitol, Lecithin, Sodium Chondroitin Sulfate, Collagen, Xanthan gum, Folic Acid, Caprylyl Glycol, Disodium EDTA, Glyceryl Caprylate, Methylparaben, Phenoxyethanol.

Moreover, there is a commercial product intended for radiation skin, of the following composition (INCI): Aqua, Octocrylene, Dibutyl Adipate, Butyl Methoxydibenzoylmethane, Ethylhexyl Triazone, Glycerin, Caprylyl Methicone, Cetearyl Alcohol, Glyceryl Stearate, Glyceryl Stearate Citrate, Butyrospermum Parkii (Shea) Butter, Caprylyl Glycol, Helianthus Annuus Seed Oil Unsaponifiables, Betaglucan, Artemia Extract, Panthenol, Tocopheryl Acetate, Allantoin, Cetearyl Ethylhexanoate, Isopropyl Myristate, Neopentyl Glycol Diheptanoate, Arginine, Polyester-7, Carbomer, Disodium Phosphate.

Additionally, there is a product commercially available, of the following composition (INCI): Aqua, Olea Europaea Fruit Oil, Sorbitan Olivate, Cetearyl Olivate, Glycerin, Panthenol 75%, Isostearyl Neopentanoate, Myreth-3 Myristate, Scutellaria Baicalensis Root Extract, Sorbeth-30, Squalane, Silybum marianum extract, Carbomer, Sodium hydroxymethylglycinate 50%, Menthol, Sodium hyaluronate.

And also, there is a product commercially available, of the following composition (INCI): Aqua, Olea Europea Oil, Myristyl Myristate, Maleated Soybean Oil, Cetearyl Olivate, Sorbitan Olivate, Glycerin, Panthenol, Polygonum cuspidatum extract, Silybum marianum fruit extract, S 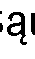 ualene, Carbomer, Sodium Hydroxymethylglycinate, Menthol.

WO 2013/016257 discloses composition for use in the treatment of radiation-induced skin damage, such as UV damage. The composition includes a botanical antioxidant extract blend including a first antioxidant botanical extract including at least one hydroxycinnamic acid, and at least one additional antioxidant botanical extracts including at least one antioxidant selected from the group consisting of vitamins, stilbenoids, curcumininoids, tannins, flavones, fiavonols, flavan-3-ols, flavanones, anthocyanidins, anthocyanins, isoflavones, flavanonols, proanthocyanidins, dihydroxybenzoic acids, and pyridine alkaloids. The antioxidant extract blend of the invention does not comprise turmerones, especially ar-turmerone, alpha-turmerone and beta-turmerone, and exemplary compositions do not contain combination of hyaluronic acid, or a salt thereof, and/or dextran sulfate, and isostearyl isostearate and/or oleyl erucate. The application is also silent about efficacy of the exemplary compositions.

However, none of the known products provides comprehensive prevention or treatment, which must involve anti-inflammatory effects associated with formation of free radicals and protection of sensitive skin from irritation, and on the other ― regeneration of the barrier function of the skin by restoring the lipid layer and providing intensive skin moisture.

Therefore, there is a need to develop such a skin composition which will meet the above mentioned criteria and which will have strong preventive and/or therapeutic action.

This objective is accomplished by the skin composition of the present invention.

The subject matter of the invention is a skin composition comprising:
a) a mixture of ar-turmerone, alpha-turmerone and beta-turmerone,
b) hyaluronic acid, or a salt thereof, and/or dextran sulfate, and
c) isostearyl isostearate and/or oleyl erucate.

A mixture of ar-turmerone, alpha-turmerone and beta-turmerone is used, such as, for example, a commercially available mixture TEGO^{®} Turmerone by Evonik. It is a distilled fraction of turmeric oil extracted from the root of *Curcuma longa* by supercritical carbon dioxide.

Mixture of turmerones has a strong anti-radical action. Therefore, this ingredient cures inflammation caused by free radicals formed in tissues due to ionising radiation. Its exceptional effectiveness results from excellent ability to penetrate the epidermis and to reach deeper layers of the skin. The action of typically used ingredients of such products, like milk thistle extract, is limited to the upper layer of epidermis.

Hyaluronic acid or a salt thereof is commercially available as, for example, HyaCare (sodium hyaluronate) by Evonik or HYALURONIC ACID-BT (hyaluronic acid) by DSM Nutritional Products. Hyaluronic acid or a salt thereof has strong moisturising and soothing effects. Due to their antistatic properties, they smooth out exfoliating epidermis, which is of particular importance for skin care in the case of dermatitis grade 2 or higher as per RTOG/EORTC.

Dextran sulfate is commercially available as, for example, Dextralip 10C by Safic-Alcan. It is an anti-inflammatory agent because it inhibits the production of prostaglandins in tissues affected by inflammation. Moreover, it demonstrates anti-radical action and strong moisturising properties.

Isostearyl isostearate is commercially available as, for example, Crodamol ISIS by Corda. Isostearyl isostearate has a beneficial effect on the structure of lipids in the dermis. A burn caused by ionising radiation or a thermal burn destabilises the structure of the lipid layer in the skin. The skin becomes more permeable to chemical agents and microorganisms and, in consequence, it is sensitive and susceptible to infections. Owing to the application of isostearyl isostearate, the skin can regain its barrier properties because this ingredient has been shown to promote the rhomboidal arrangement of lipid structures, which corresponds to their arrangement in the healthy skin, as opposed to the hexagonal arrangement in the skin in which the lipid barrier has been compromised. (G. Pennick, S. Harrison, D. Jones and A.V. Rawlings International Journal of Cosmetic Science, 2010, 32, 304-312).

Oleyl erucate is commercially available, for example as Tegosoft OER by Evonik. It has very good greasing and protective properties. This ingredient effectively protects sensitive skin against irritations.

The composition of the present invention may have any form suitable for application on the skin.

In an advantageous embodiment, the composition of the present invention has the form of an oil-in-water emulsion.

According to the present invention, the ingredients can be admixed to the pre-prepared oil-in-water emulsion base, or they can be used as appropriate phases or phase ingredients in the process of production of this emulsion. Methods for producing such appropriate emulsion bases and general methods of producing emulsions can be found, for example, in Flick, E. W., Cosmetic and toiletry formulations, Noyes Publications (1992).

This emulsion may contain water, emollient, humectant, emulsifier and a consistency-forming substance.

Examples of emollients include: vegetable oils, esters of fatty acids and higher fatty alcohols, esters of fatty acids and short-chain alcohols. Emollients that are advantageous and are in particular used in the composition of the present invention include: synthetic caprylic/capric triglyceride (trade name Tegosoft CT), isohexadecane (trade name Arlamol HD), ethyl-hexyl stearate (trade name Cetiol 868), isostearyl isostearate (trade name Crodamol ISIS), mixture of C13-C15 alcohol benzoates (trade name Crodamol AB), oleyl erucate (trade name Tegosoft OER) and rapeseed oil (Brassica Campestris (Rapeseed) Seed Oil).

Examples of humectants that can be used in the composition of the present invention include pentylene glycol (trade name Neofect PEN), glycerol, 1,3-propanediol (trade name Zemea), panthenol (trade name Panthenol) and xylitol (trade name Xylitol).

Furthermore, examples of emulsifiers that can be used in a composition of the present invention include glycerol stearate (trade name Cutina GSM SE) or polyglyceryl-3 methylglucose distearate (trade name Tego Care 450).

Examples of consistency-forming substances that can be used in a composition of the present invention include stearyl alcohol (trade name Tego Alcanol 18) or cetearyl alcohol. However, in an advantageous embodiment, the composition of the present invention contains only a small amount or no consistency-forming substance, which ensures a suitably low viscosity of the composition of the present invention.

Preferably, the skin composition of the present invention contains
a) a mixture of ar-turmerone, alpha-turmerone and beta-turmerone at 0.1 to 0.5% w/w of the whole composition,
b) hyaluronic acid, or a salt thereof, at 0.05 to 0.3% w/w of the whole composition and/or dextran sulfate at 0.1 to 1.0% w/w of the whole composition, and
c) isostearyl isostearate at 1 do 10% w/w of the whole composition and/or oleyl erucate at 1 to 10% w/w of the whole composition.

Preferably, the skin composition of the present invention contains both hyaluronic acid and dextran sulfate.

Preferably, the skin composition of the present invention contains both isostearyl isostearate and oleyl erucate.

In a preferable embodiment, the skin composition of the present invention additionally contains at least one detergent; whereby, the invention provides a skin detergent composition. Addition of a detergent to the composition will give it cleansing properties. A detergent to be added to the composition of the present invention should, on the one hand, be sufficiently mild and not irritant to the affected skin, and on the other ― ensure its proper cleansing. Examples of such detergents include diethylhexyl sodium sulfosuccinate (trade name Tego Sulfosuccinate DO 75), cocoamidopropyl betaine (trade name Tego Betain F 50) or disodium laureth sulfosuccinate (trade name Rewopol SB FA 30 B) and a mixture of water, sodium lauroyl methyl isethionate, sodium lauroamphoacetate and cocamide MIPA (trade name Iselux^{®} SLC by Innospec). A particularly preferable detergent is a mixture of water, sodium lauroyl methyl isethionate, sodium lauroamphoacetate and cocamide MIPA.

Additionally, in a preferable embodiment, the skin composition of the present invention contains a colloidal silver. Thereby, the invention provides a skin composition with silver.

A colloidal silver is known to have antimicrobial properties, therefore, addition of this ingredient to the skin composition of the present invention will give it antimicrobial properties. Such an effect is particularly desired in radiation-induced skin reactions of grade 2 or higher. In such cases, desquamation can be accompanied by local microbial infections. Such a composition will be particularly suitable in the case of radiation-induced skin reactions of grade 2 or higher. Preferably, the colloidal silver can be used is the form of microsilver, for example, a commercially available product MicroSilver BG^{™} having solid silver particles with a size in the micrometer range. Silver particles are also available with a size in the nanometer range. The silver in the nanometer range has the disadvantage of the black color. In our studies we used microsilver that is whitish.

Compositions of the invention as emulsions can be presented as various consistencies. For example, emulsion can have the form of an ointment, a cream, a lotion or a spray. In an advantageous embodiment, the skin composition of the present invention has the form of a spray. Suitable sprays can be manufactured using the techniques presented in Flick , E. W., Cosmetic and toiletry formulations, Noyes Publications (1992). Preparing the said composition as a spray, on the one hand, makes its application on the skin easier and, on the other hand, low viscosity of such a composition prevents additional skin irritation which may occur when preparations of high viscosity are applied.

The present invention also relates to the skin composition of the present invention mentioned above or a spray which contains the skin composition of the present invention for use in the prevention and/or treatment of radiation-induced skin reactions.

The skin composition of the present invention can be applied as a preventive measure, i.e. before any adverse effects of radiation appear; preferably, however, a treatment with the composition of the present invention can be started simultaneously with radiation therapy.

The skin composition of the present invention can be applied as a therapeutic measure, i.e. after the onset of the first signs of radiation toxicities.

The skin composition can be applied independently, i.e. only the composition in the form of a detergent-free skin care spray or only the composition containing a detergent for cleansing the skin can be used. However, preferably, both the detergent-free and detergent-containing composition can be used.

The skin composition of the present invention can be used in the following manner. The skin affected by the radiation-induced reaction is first washed with tepid water with the skin composition of the present invention comprising a detergent; subsequently, an appropriate amount of the detergent-free skin composition is applied on the dry skin. Preferably, the latter composition of the present invention mentioned above has the form of a spray.

The skin composition of the present invention has a lot of advantages. First of all, it provides a comprehensive therapy, which, on the one hand, involves anti-inflammatory effects associated with the appearance of free radicals owing to the presence of a mixture of ar-turmerone, alpha-turmerone and beta-turmerone and, possibly, dextran sulfate, and the protection of sensitive skin from irritation owing to the presence of oleyl erucate, and on the other ― regeneration of the barrier function of the skin by restoring the lipid layer owing to the presence of isostearyl isostearate or oleyl erucate and intensive moisturisation of the skin owing to the addition of hyaluronic acid or a salt thereof and/or dextran sulfate.

Spray comprising the composition of the present invention enables easy application of the composition of the present invention as well as easy and convenient spreading of the composition without additional irritation of the affected skin area.

### Examples

### Formulas for burned skin

### Example 1. Spray comprising the skin composition

Phase A is combined with Phase B and the whole is mixed at a room temperature with a frame paddle stirrer for 5 minutes. Phases C, D and E are added sequentially to the resulting emulsion and the emulsion is stirred for 2 minutes after addition of each phase. Phase F is added at the end and the whole is stirred for 5 minutes. The resulting emulsion is left overnight and then it is portioned and packaged.

| **Trade name** | **INCI name** | % |
|---|---|---|
| **Phase A** | | |
| Tego Care LTP | Sorbitan Laurate, Polyglyceryl-4 Laurate, Dilauryl Citrate | 2.0 |
| Tegosoft CT | Caprylic/Capric Triglycerides | 7.5 |
| Crodamol ISIS | Isostearyl Isostearate | 5.0 |
| Tegosoft OER | Oleyl Erucate | 2.0 |

| **Phase B** | | |
|---|---|---|
| Water | Water | Up to 100 |

| **Phase C** | | |
|---|---|---|
| Sk-Influx | Ceramide 3, Ceramide 6 II, Ceramide 1, | 3.0 |
| | Phytosphingosine, Cholesterol, Sodium Lauroyl | |
| | Lactylate, Carbomer, Xanthan Gum | |

| **Phase D** | | |
|---|---|---|
| Tego Turmerone | Curcuma Longa (Turmeric) Root Extract | 0.5 |

| **Phase E** | | |
|---|---|---|
| Glycerin | Glycerin | 1.8 |
| Dextralip 10C | Sodium Dextran Sulfate | 0.1 |
| HyaCare | Sodium Hyaluronate | 0.2 |
| Panthenol | Panthenol | 1.0 |
| Tego Carbomer 141 | Carbo mer | 0.1 |
| Tego Carbomer 341 ER | Acrylates / C10-30 Alkyl Acrylate Crosspolymer | 0.1 |
| Methyl Paraben | Methyl Paraben | 0.15 |
| Propyl Paraben | Propyl Paraben | 0.15 |

| **Phase F** | | |
|---|---|---|
| Sodium Hydroxide 10% | Sodium Hydroxide | 0.3 |

### Example 2 Spray comprising the skin composition

Phases A and B are heated up to 75°C in separate containers. The Phases are combined and homogenised for one minute. Next, the resulting emulsion is stirred with a mechanical stirrer until it cools down to 40°C. Phases C, D and E are added sequentially to the emulsion. Phase F is added when the emulsion has cooled down to 25°C and the whole is stirred for 5 minutes.

| **Trade name** | **INCI name** | % |
|---|---|---|
| **Phase A** | | |
| TEGO Care PBS 6 | Polyglyceryl-6 Stearate (and) Polyglyceryl-6 Behenate | 3.0 |
| TEGIN M Pellets | Glyceryl Stearate | 0.5 |
| TEGO Alkanol 18 | Stearyl Alcohol | 0.5 |
| Tegosoft CT | Caprylic/Capric Triglycerides | 7.5 |
| Crodamol ISIS | Isostearyl Isostearate | 5.0 |
| Tegosoft OER | Oleyl Erucate | 2.0 |

| **Phase B** | | |
|---|---|---|
| Water | Water | Up to 100 |

| **Phase C** | | |
|---|---|---|
| Sk-Influx | Ceramide 3, Ceramide 6 II, Ceramide 1, | 3.0 |
| | Phytosphingosine, Cholesterol, Sodium Lauroyl | |
| | Lactylate, Carbomer, Xanthan Gum | |

| **Phase D** | | |
|---|---|---|
| Tego Turmerone | Curcuma Longa (Turmeric) Root Extract | 0.5 |
| Phase E | | |
| Glycerin | Glycerin | 2.0 |
| Dextralip 10C | Sodium Dextran Sulfate | 0.5 |
| Panthenol | Panthenol | 1.0 |
| Tego Carbomer 341 ER | Acrylates / C10-30 Alkyl Acrylate Crosspolymer | 0.2 |
| Methyl Paraben | Methyl Paraben | 0.15 |
| Propyl Paraben | Propyl Paraben | 0.15 |

| **Phase F** | | |
|---|---|---|
| Sodium Hydroxide 10% | Sodium Hydroxide | 0.3 |

### Example 3 Spray comprising the skin composition

Phases A and B are heated up to 75°C in separate containers. The Phases are combined and homogenised for one minute. Next, the emulsion is stirred with a mechanical stirrer until it cools down to 40°C. Phases C, D and E are added sequentially to the emulsion. Phase F is added when the emulsion has cooled down to 25°C and the whole is stirred for 5 minutes.

| **Trade name** | **INCI name** | % |
|---|---|---|
| **Phase A** | | |
| Montanov 82 | Cetearyl Alcohol and Coco-Glucoside | 2.0 |
| Tegosoft CT | Caprylic/Capric Triglycerides | 7.5 |
| Crodamol ISIS | Isostearyl Isostearate | 5.0 |
| Tegosoft OER | Oleyl Erucate | 2.0 |

| **Phase B** | | |
|---|---|---|
| Water | Water | Up to 100 |

| **Phase C** | | |
|---|---|---|
| Sk-Influx | Ceramide 3, Ceramide 6 II, Ceramide 1, | 3.0 |
| | Phytosphingosine, Cholesterol, Sodium Lauroyl | |
| | Lactylate, Carbomer, Xanthan Gum | |

| **Phase D** | | |
|---|---|---|
| Tego Turmerone | Curcuma Longa (Turmeric) Root Extract | 0.5 |

| **Phase E** | | |
|---|---|---|
| Glycerin | Glycerin | 2.0 |
| HyaCare | Sodium Hyaluronate | 0.05 |
| Panthenol | Panthenol | 1.0 |
| Carbo pol | Carbo mer | 0.2 |
| Methyl Paraben | Methyl Paraben | 0.15 |
| Propyl Paraben | Propyl Paraben | 0.15 |

| **Phase F** | | |
|---|---|---|
| Sodium Hydroxide 10% | Sodium Hydroxide | 0.3 |

### Example 4 Spray comprising the skin composition

Phases A and B are heated up to 75°C in separate containers. The Phases are combined and homogenised for one minute. Next, the emulsion is stirred with a mechanical stirrer until it cools down to 40°C. Phases C, D and E are added sequentially to the emulsion. Phase F is added when the emulsion has cooled down to 25°C and the whole is stirred for 5 minutes.

| **Trade name** | **INCI name** | % |
|---|---|---|
| **Phase A** | | |
| Montanov 82 | Cetearyl Alcohol and Coco-Glucoside | 2.0 |
| TIMECODE | Palmitoyl Glycine | 1.0 |
| Tegosoft CT | Caprylic/Capric Triglycerides | 7.5 |
| Crodamol ISIS | Isostearyl Isostearate | 5.0 |
| Tegosoft OER | Oleyl Erucate | 2.0 |

| **Phase B** | | |
|---|---|---|
| Water | Water | Up to 100 |

| **Phase C** | | |
|---|---|---|
| Sk-Influx | Ceramide 3, Ceramide 6 II, Ceramide 1, | 3.0 |
| | Phytosphingosine, Cholesterol, Sodium Lauroyl | |
| | Lactylate, Carbomer, Xanthan Gum | |

| **Phase D** | | |
|---|---|---|
| Tego Turmerone | Curcuma Longa (Turmeric) Root Extract | 0.5 |

| **Phase E** | | |
|---|---|---|
| Glycerin | Glycerin | 2.0 |
| Dextralip 10C | Sodium Dextran Sulfate | 0.1 |
| Panthenol | Panthenol | 1.0 |
| Tego Carbomer 141 | Carbo mer | 0.1 |
| Tego Carbomer 341 ER | Acrylates / C10-30 Alkyl Acrylate Crosspolymer | 0.1 |
| Methyl Paraben | Methyl Paraben | 0.15 |
| Propyl Paraben | Propyl Paraben | 0.15 |

| **Phase F** | | |
|---|---|---|
| Sodium Hydroxide 10% | Sodium Hydroxide | 0.3 |

### Example 5 The skin composition comprising a detergent

Phase A is heated up to 80°C and stirred with a rotor stator stirrer. Phase B is heated up to 80°C and added to Phase A. The phases are stirred with a rotor stator stirrer until they cool down to 40°C. Next, the whole is stirred with a stirrer and Phase C is added. Phase D is added when the mixture has cooled down to 25°C.

| **Trade name** | **INCI name** | % |
|---|---|---|
| **Phase A** | | |
| Water | Water | Up to 100 |
| Lanette O | Cetearyl Alcohol | 7.5 |
| Montanov 82 | Cetearyl Alcohol and Coco-Glucoside | 2.5 |
| Xylitol | Xylitol | 3.0 |

| **Phase B** | | |
|---|---|---|
| Rapeseed oil | Brassica Campestris (Rapeseed) Seed Oil | 3.0 |

| **Phase C** | | |
|---|---|---|
| Tego Turmerone | Curcuma Longa (Turmeric) Root Extract | 0.5 |
| Crodamol ISIS | Isostearyl Isostearate | 5.0 |

| **Phase D** | | |
|---|---|---|
| Glycerin | Glycerin | 2.0 |
| Dextralip 10C | Sodium Dextran Sulfate | 0.1 |
| Iselux^{®} | Water (and) Sodium Lauroyl Methyl Isethionate (and) Sodium Lauroamphoacetate (and) Cocamide MIPA | 1.0 |
| GEOGARD 221 | Dehydroacetic Acid and Benzyl Alcohol | 0.6 |

### Example 6 The skin composition comprising a detergent

Phase A is heated up to 80°C and stirred with a mechanical stirrer until the mixture cools down to 50°C. Next, Phases B and C are added, while the whole is stirred slowly. Phases D, E and F are added sequentially after 5 minutes of stirring.

| **Trade name** | **INCI name** | % |
|---|---|---|
| **Phase A** | | |
| Water | Water | Up to 100 |
| Montanov 82 | Cetearyl Alcohol and Coco-Glucoside | 2.0 |
| ANTIL 120 PLUS | PEG-120 Methylglucose Dioleate | 4.1 |
| Lactic Acid 12% | Lactic Acid | 0.1 |

| **Phase B** | | |
|---|---|---|
| Sodium Lauryl Ether Sulfate 28% | Sodium Lauryl Ether Sulfate | 22.0 |

| **Phase C** | | |
|---|---|---|
| Tego Turmerone | Curcuma Longa (Turmeric) Root Extract | 0.5 |
| Crodamol ISIS | Isostearyl Isostearate | 2.0 |

| **Phase D** | | |
|---|---|---|
| Glycerin | Glycerin | 5.0 |
| HyaCare | Sodium Hyaluronate | 0.05 |

| **Phase E** | | |
|---|---|---|
| TEGO Betain P 50 C | Cocamidopropyl Betaine | 7.0 |

| **Phase F** | | |
|---|---|---|
| GEOGARD 221 | Dehydroacetic Acid and Benzyl Alcohol | 0.6 |

### Example 9 Spray comprising the skin composition with silver

The spray is prepared by admixing 0.2% w/w of colloidal silver (MicroSilver BG^{™}) to the composition prepared as per Example 1 above.

### Example 10 The skin composition comprising a detergent and with silver

A skin composition comprising a detergent and comprising silver is prepared by admixing 0.1% w/w of colloidal silver (MicroSilver BG^{™}) to the composition prepared as per Example 5 above.

### Example 11 Results of comparative studies on preventive action

Twelve people aged 22-67 years with head and neck cancers, who received radiation therapy to the neck for at least 28 days, were selected for comparative studies. They were divided into three groups, four people in each. The first group used the composition of the present invention with a detergent as per Example 5 and the detergent-free composition as per Example 1, the second group used placebo compositions similar to those in Examples 5 and 1 but without turmerones, hyaluronate, dextran sulfate, isostearyl isostearate or oleyl erucate. The third group applied a commercial product of the following composition (INCI): Aqua, Paraffinum Liquidum, Isododecane, Pentylene Glycol, Panthenol, Glycerin, Cetyl PEG/PPG-10/1 Dimethicone, Magnesium Sulfate, Polyglyceryl-4 Isostearate, Cera Alba (Beeswax), Sorbitol, Lecithin, Sodium Chondroitin Sulfate, Collagen, Xanthan gum, Folic Acid, Caprylyl Glycol, Disodium EDTA, Glyceryl Caprylate, Methylparaben, Phenoxyethanol.

Application of the cleansing composition of the present invention with a detergent as per Example 5 was started on the first day of radiation therapy, followed by application of the skin care composition of the present invention without a detergent as per Example 1. Corresponding placebo compositions were applied in group two. The third group applied a commercial cosmetic product of the following composition (INCI): Aqua, Paraffinum Liquidum, Isododecane, Pentylene Glycol, Panthenol, Glycerin, Cetyl PEG/PPG-10/1 Dimethicone, Magnesium Sulfate, Polyglyceryl-4 Isostearate, Cera Alba (Beeswax), Sorbitol, Lecithin, Sodium Chondroitin Sulfate, Collagen, Xanthan gum, Folic Acid, Caprylyl Glycol, Disodium EDTA, Glyceryl Caprylate, Methylparaben, Phenoxyethanol.

Skin condition at the irradiated site was assessed visually on days 7, 14 and 28. The results for the therapeutic group, for the placebo group and for the reference group were collected in the following table.

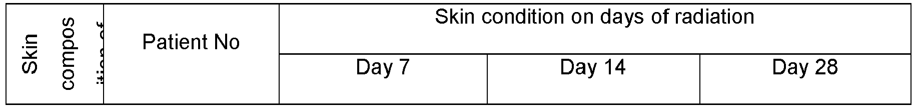

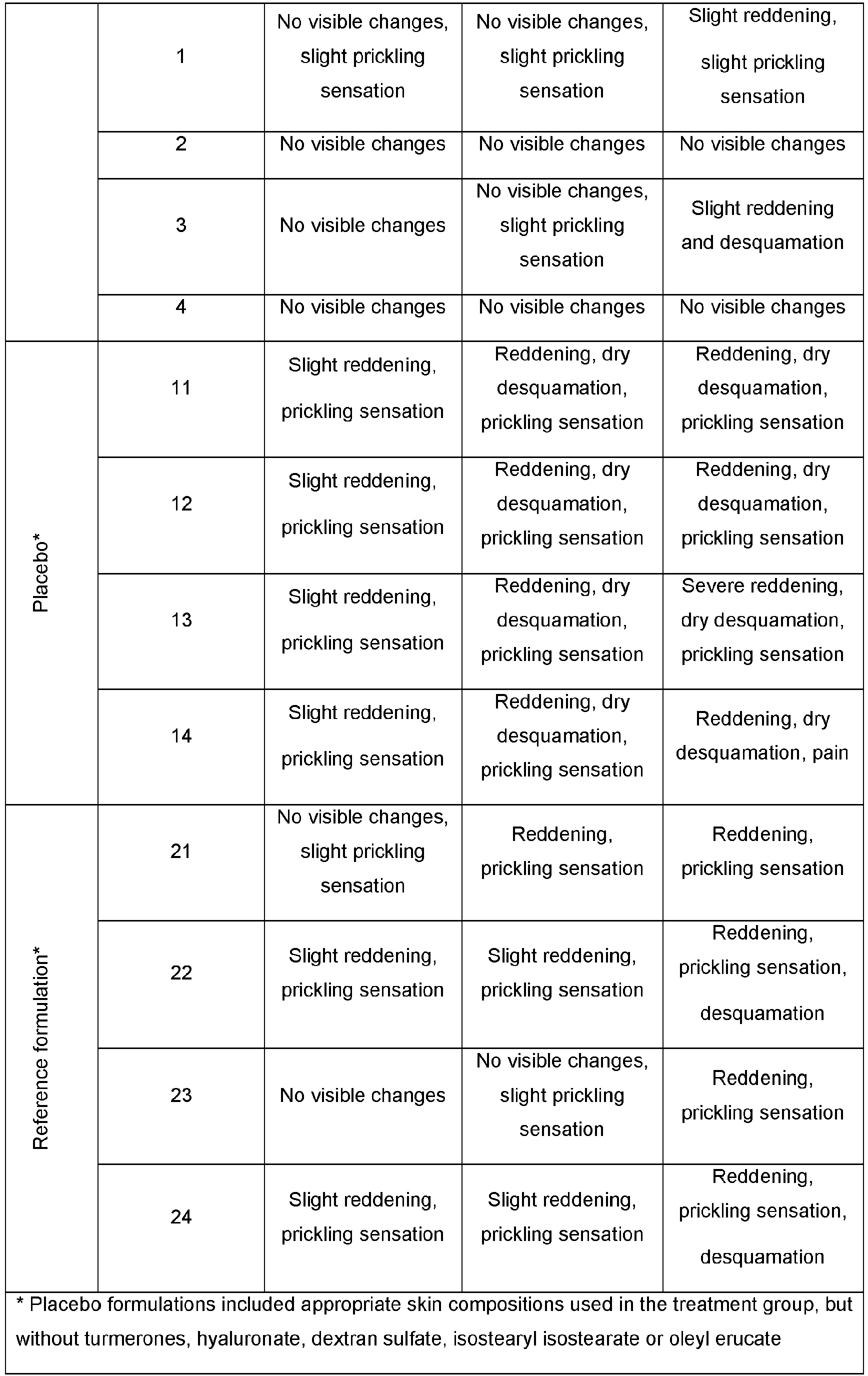

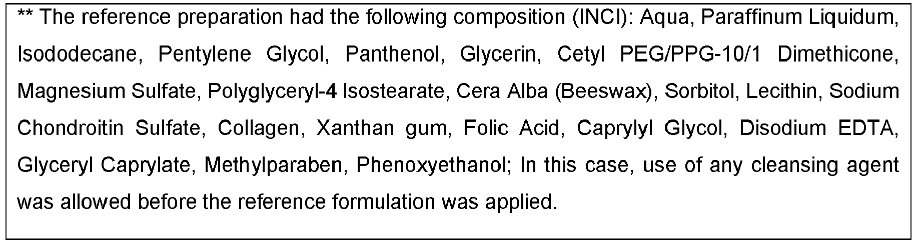

The results presented above clearly show that skin compositions of the invention very effectively prevent radiation-induced skin reactions.

Visible changes in the form of slight reddening were observed only in some of the individuals who applied the composition of the present invention, and only at a late stage of the radiotherapy cycle (day 28 of observation). Only slight prickling sensation at the irradiated site on the skin was noted on day 14 in some of the subjects who applied the compositions of the invention.

On the other hand, slight reddening appeared on day 7 of radiation therapy in the placebo group and the symptoms exacerbated on consecutive days, so that severe reddening and dry desquamation were observed in all subjects on day 28.

No visible changes were observed after 7 days in some people who applied the reference formulation; however, reddening appeared in all subjects in this group after 28 days.

### Example 12 Results of comparative studies on therapeutic action

Fifteen people aged 34-67 years with head and neck cancer, who received radiation therapy to the neck, were selected for comparative studies. They were divided into two groups: therapeutic and control, with 10 and 5 people, respectively. The first group applied the composition of the present invention comprising a detergent as per Example 5 and the detergent-free composition as per Example 1, the second group applied a commercial cosmetic product of the following composition (INCI): Aqua, Paraffinum Liquidum, Isododecane, Pentylene Glycol, Panthenol, Glycerin, Cetyl PEG/PPG-10/1 Dimethicone, Magnesium Sulfate, Polyglyceryl-4 Isostearate, Cera Alba (Beeswax), Sorbitol, Lecithin, Sodium Chondroitin Sulfate, Collagen, Xanthan gum, Folic Acid, Caprylyl Glycol, Disodium EDTA, Glyceryl Caprylate, Methylparaben, Phenoxyethanol. The effects of application of the composition as per Example 5 and 1 were compared with those of a therapeutic ointment ARGOSULFAN in patients with severe skin reactions and compromised skin integrity.

Application of the cleansing composition of the present invention comprising a detergent as per Example 5, followed by application of the skin care composition of the present invention in the form of a spray as per Example 1, was started on the day when the first visible symptoms of a radiation-induced skin reaction appeared.

An assessment of the therapy with the compositions of the invention is presented in the following table.

| Patient No | Condition of irradiated skin | Effect of therapy with the composition of the present invention |
|---|---|---|
| GF34F | Severe reddening, pain | Skin condition improved after the first application. Reddening and pain diminished. Slight skin irritation was observed after irradiation was completed. |
| AM41F | Slight reddening, prickling sensation | Skin condition improved after the first application. Reddening diminished. No symptoms of a skin reaction were observed after radiation was completed. |
| TK55M | Severe reddening, pain, skin cracking | The formulation was tolerated well. Skin condition improved after several days of application. Only slight skin reaction was observed after radiation was completed. The symptoms of skin reaction resolved after another week of treatment. |
| BR61F | Reddening, desquamation, pain | The formulation was tolerated well. Skin condition improved after the first application. Reddening and pain diminished. Slight skin irritation was observed after irradiation was completed. The symptoms of skin reaction resolved after another week of treatment. |
| IN47M | Reddening, prickling sensation | The symptoms of skin reaction were alleviated on the first day of application. Slight skin irritation was observed after irradiation was completed. The symptoms of skin reaction resolved after another week of treatment. |
| BT59F | Severe reddening, pain, skin cracking | The formulation was tolerated well. Skin condition improved after the first application. Reddening and pain diminished. Slight skin irritation was observed after irradiation was completed. The symptoms of skin reaction resolved after another week of treatment. |
| KK49M | Reddening, desquamation, pain | The preparation was tolerated very well by the patient. Skin condition improved after the first application. Reddening and pain diminished. The symptoms of skin reaction resolved after another week from completion of radiation. |
| MT52M | Severe reddening, pain, skin cracking | The formulation was tolerated well. Skin condition improved after several days of application. Only slight skin reaction was observed after radiation was completed. The symptoms of skin reaction resolved after another week of treatment. |
| RT58F | Severe reddening, pain, skin cracking, ulceration | The formulation was tolerated well. Pain diminished. Symptoms of infection and severe reaction resolved after several days. No symptoms of a skin reaction were observed after the treatment was completed. |
| ES63F | Ulceration, pain | The formulation was tolerated well. Pain diminished. Symptoms of infection and severe reaction resolved after several days. No symptoms of a skin reaction were observed after the treatment was completed. |

An assessment of the therapy with commercial formulations is presented in the following table.

| Patient No | Condition of irradiated skin | Effect of treatment |
|---|---|---|
| MN46F | Reddening, desquamation, pain | Cosmetic formulation |
| | | The formulation was tolerated well. Skin condition improved after several days of application. Skin reaction was still observed after radiation was completed. Slight reddening and prickling sensation persisted after another week of application. |

| Patient No | Condition of irradiated skin | Effect of treatment |
|---|---|---|
| UB52F | Slight reddening, prickling sensation | Cosmetic formulation |
| | | Skin condition improved after several days of application. Skin reaction was still observed after radiation was completed. Slight reddening persisted after another week of application. |
| KL61 M | Severe reddening, pain, skin cracking | ARGOSULFAN |
| | | Symptoms of infection resolved. Pain did not diminish. Skin reaction diminished 2 weeks after radiation was completed. |
| GM47M | Ulceration, pain | ARGOSULFAN |
| | | Symptoms of infection resolved. Pain diminished. Skin reaction diminished 2 weeks after radiation was completed. |
| KR67F | Reddening, prickling sensation | Cosmetic formulation |
| | | The formulation was tolerated well. Skin condition improved after several days of application. Skin reaction was still observed after radiation was completed. Slight reddening and prickling sensation persisted after another week of application. |

Skin compositions as per Example 5 and 1 were tolerated well by the patients. Symptoms were alleviated after the first application in all the cases. Due to its low viscosity, emulsion as per Example 1 did not cause any irritations when rubbed into the skin. ARGOSULFAN treats skin infections in a similar manner to the composition as per Example 1. However, it does not have a soothing effect and, according to the patients' reports, it is applied worse on the skin surface.

No recurrence of skin reaction symptoms was observed after the treatment with the skin compositions as per Example 5 and 1 was completed.

## Claims

1. A skin composition comprising:
a) a mixture of ar-turmerone, alpha-turmerone and beta-turmerone,
b) hyaluronic acid, or a salt thereof, and/or dextran sulfate, and
c) isostearyl isostearate and/or oleyl erucate.

2. A composition according to claim 1, **characterized in that** it has the form of an oil-in-water emulsion.

3. A composition according to claim 1 or 2, **characterized in that** it comprises
a) a mixture of ar-turmerone, alpha-turmerone and beta-turmerone at 0.1 to 0.5% w/w of the whole composition,
b) hyaluronic acid, or a salt thereof, at 0.05 to 0.3% w/w of the whole composition and/or dextran sulfate at 0.1 to 1.0% w/w of the whole composition, and
c) isostearyl isostearate at 1 do 10% w/w of the whole composition and/or oleyl erucate at 1 to 10% w/w of the whole composition.

4. A composition according to any of the above claims **characterized in that** it comprises hyaluronic acid and dextran sulfate.

5. A composition according to any of the above claims **characterized in that** it comprises isostearyl isostearate and oleyl erucate.

6. A composition according to any of the above claims **characterized in that** it comprises additionally at least one detergent.

7. A composition according to claim 6, wherein the detergent is a mixture of water, sodium lauroyl methyl isethionate, sodium lauroamphoacetate and cocamide MIPA.

8. A composition according to any of the above claims **characterized in that** it additionally contains colloidal silver.

9. A composition according to claim 8, **characterized in that** it comprises a colloidal silver at 0.1 to 0.3% w/w of the whole composition.

10. Spray containing a composition according to any of claims 1 to 9.

11. A composition according to any of claims 1-9 or spray according to claim 10 for use in the prevention and/or treatment of radiation-induced skin reactions.

## Patentansprüche

1. Zusammensetzung für die Haut, umfassend:
a) eine Mischung von ar-Turmeron, alpha-Turmeron und beta-Turmeron,
b) Hyaluronsäure oder ein Salz davon, und/oder Dextransulfat, und
c) Isostearylisostearat und/oder Oleylerucat.

2. Zusammensetzung nach Anspruch 1, **dadurch gekennzeichnet, dass** sie ein form von Öl-in-Wasser-Emulsion hat.

3. Zusammensetzung nach Anspruch 1 oder 2, **dadurch gekennzeichnet, dass** sie:
a) eine Mischung von ar-Turmeron, alpha-Turmeron und beta-Turmeron in einer Konzentration von 0,1 bis 0,5 Gew.-% der gesamten Zusammensetzung,
b) Hyaluronsäure oder ein Salz davon in einer Konzentration von 0,05 bis 0,3 Gew.- % der gesamten Zusammensetzung, und/oder Dextransulfat in einer Konzentration von 0,1 bis 1,0 Gew.-% der gesamten Zusammensetzung, und
c) Isostearylisostearat in einer Konzentration von 1 bis 10 Gew.-% der gesamten Zusammensetzung und/oder Oleylerucat in einer Konzentration von 1 bis 10 Gew.-% der gesamten Zusammensetzung,
umfasst.

4. Zusammensetzung nach einem der obigen Ansprüche, **dadurch gekennzeichnet, dass** sie Hyaluronsäure und Dextransulfat umfasst.

5. Zusammensetzung nach einem der obigen Ansprüche, **dadurch gekennzeichnet, dass** sie Isostearylisostearat und Oleylerucat umfasst.

6. Zusammensetzung nach einem der obigen Ansprüche, **dadurch gekennzeichnet, dass** sie zusätzlich mindestens ein Detergens umfasst.

7. Zusammensetzung nach Anspruch 6, wobei das Detergens eine Mischung von Wasser, Natriumlauroylmethylisethionat, Natriumlauroamphoacetat und Cocamid-MIPA ist.

8. Zusammensetzung nach einem der obigen Ansprüche, **dadurch gekennzeichnet, dass** sie zusätzlich kolloidales Silber enthält.

9. Zusammensetzung nach Anspruch 8, **dadurch gekennzeichnet, dass** sie zusätzlich ein kolloidales Silber in einer Konzentration von 0,1 bis 0,3 Gew.-% der gesamten Zusammensetzung enthält.

10. Spray, enthaltend eine Zusammensetzung nach einem der Ansprüche 1 bis 9.

11. Zusammensetzung nach einem der Ansprüche 1-9 oder Spray nach Anspruch 10 zur Verwendung bei der Vorbeugung und/oder Behandlung von strahlungsinduzierten Hautreaktionen.

## Revendications

1. Composition pour la peau comprenant :
a) un mélange de l'ar-turmérone, de l'alpha-turmérone et de la béta-turmérone,
b) l'acide hyaluronique, ou un sel de celui-ci, et/ou le sulfate de dextrane, et
c) l'isostéarate d'isostéaryle et/ou l'érucate d'oléyle.

2. Composition selon la revendication 1, **caractérisée en ce qu'**elle a la forme d'une émulsion l'huile dans l'eau.

3. Composition selon la revendication 1 ou 2, **caractérisée en ce qu'**elle comprend
a) un mélange de l'ar-turmérone, de l'alpha-turmérone et de la béta-turmérone en concentration de 0,1 à 0,5 % en poids de la composition totale,
b) l'acide hyaluronique ou un sel de celui-ci en concentration de 0,05 à 0,3 % en poids de la composition totale, et/ou le sulfate de dextrane en concentration de 0,1 à 1,0 % en poids de la composition totale, et
c) l'isostéarate d'isostéaryle en concentration de 1 à 10 % en poids de la composition totale et/ou l'érucate d'oléyle en concentration de 1 à 10 % en poids de la composition totale.

4. Composition selon l'une quelconque des revendications ci-dessus, **caractérisée en ce qu'** elle comprend l'acide hyaluronique et le sulfate de dextrane.

5. Composition selon l'une quelconque des revendications ci-dessus, **caractérisée en ce qu'** elle comprend l'isostéarate d'isostéaryle et l'érucate d'oléyle.

6. Composition selon l'une quelconque des revendications ci-dessus, **caractérisée en ce qu'** elle comprend en outre au moins un détergent.

7. Composition selon la revendication 6, dans laquelle le détergent est un mélange d'eau, de lauroylméthyliséthionate de sodium, de lauroamphoacétate de sodium et de cocamide MIPA.

8. Composition selon l'une quelconque des revendications ci-dessus, **caractérisée en ce qu'** elle contient en outre l'argent colloidal totale.

9. Composition selon la revendication 8, **caractérisée en ce qu'** elle comprend un argent colloïdal en concentration de 0,1 à 0,3 % en poids de la composition totale.

10. Spray contenant une composition selon l'une quelconque des revendications 1 à 9.

11. Composition selon l'une quelconque des revendications 1 à 9 ou spray selon la revendication 10 pour une utilisation dans la prévention et/ou le traitement des réactions cutanées induites par la radiation.
